# EUROPÄISCHE PATENTSCHRIFT

(11) **EP 0 585 681 B1**
(45) Veröffentlichungstag und Bekanntmachung des Hinweises auf die Patenterteilung: **20.06.2001**
(21) Anmeldenummer: 93112929.0
(22) Anmeldetag: 12.08.1993
(51) Int. Cl.: A61K 38/17

(54) **Verwendung des IL-4-Rezeptors zur Therapie, Prophylaxe und Diagnose von Infektionskrankheiten**
Use of IL-4 receptors for the therapy, prophylaxis and diagnosis of infectious diseases
Utilisation des récepteurs de IL-4 pour la thérapie, la prophylaxie et le diagnostique des maladies infectieuses

(30) Priorität: 31.08.1992 DE 4228941; 05.07.1993 DE 4322330
(43) Veröffentlichungstag der Anmeldung: 09.03.1994
(73) Patentinhaber: HOECHST AKTIENGESELLSCHAFT, 65926 Frankfurt am Main (DE)
(72) Erfinder: Enssle, Karlheinz, D-35041 Marburg-Michelbach (DE); Kurrle, Roland, D-35096 Niederweimar (DE); Lauffer, Leander, D-35075 Gladenbach (DE); Seiler, Friedrich-Robert, D-35041 Marburg (DE)

(56) Entgegenhaltungen:
- EP-A- 0 367 566
- EP-A- 0 419 091
- EP-A- 0 488 170
- WO-A-92/05698
- THE JOURNAL OF IMMUNOLOGY Bd. 144, Nr. 8 , 15. April 1990 , BALTIMORE MD, USA Seiten 3028 - 3033 C. MALISZEWSKI ET AL. 'Cytokine receptors and B cell functions.'
- THE JOURNAL OF IMMUNOLOGY Bd. 149, Nr. 2 , 15. Juli 1992 , BALTIMORE MD, USA Seiten 655 - 660 W. FANSLOW ET AL. 'Soluble forms of CD40 inhibit biologic responses of human B cells.'
- THE JOURNAL OF ALLERGY AND CLINICAL IMMUNOLOGY Bd. 91, Nr. 1(2) , Januar 1993 , ST. LOUIS MO, USA Seite 234 H. RENZ ET AL. 'Inhibition of allergen-induced and allergen-specific IgE and IgG1 production by soluble IL-4 receptor (sIL-4R).'

## Beschreibung

Die Therapie und Prophylaxe vieler, viraler, parasitärer und bakterieller Erkrankungen stellt nach wie vor ein großes Problem dar. Die Erfindung betrifft die Verwendung des IL-4-Rezeptors oder von Derivaten davon Herstellung eines Arzneimittels zur Therapie, Prophylaxe und Diagnose solcher Erkrankungen.

Bei einigen parasitären, viralen und bakteriellen Erkrankungen ist bekannt, daß in ihrem Verlauf Veränderungen in Subpopulationen von lymphozytären und monozytären Zellen auftreten. Dies betrifft zum Beispiel das vermehrte Auftreten von sogenannten T-Helfer-Zellen des Typs 2 (im folgenden TH2-Zellen genannt). T-Zellen können generell aufgrund von Oberflächenmarkern und aufgrund ihrer Funktion in Subpopulationen unterteilt werden. So tragen beispielsweise T-Helfer-Lymphozyten CD4-Oberflächenmoleküle und sezernieren nach ihrer Aktivierung Cytokine.

Analysen des Cytokin-Musters von klonierten T-Helferzellen aus gesunden Mäusen oder mit allogenen Zellen stimulierten Mäusen ergaben, daß diese Zellen Interleukin-2, Interleukin-4, Gamma-Interferon, Interleukin-5, Interleukin-6, Interleukin-10 und Lymphotoxin produzieren (T-Helfer-Zellen vom sogenannten THO-Typ).

Nach Stimulation von Mäusen, zum Beispiel mit dem bakteriellen Antigen Brucella abortus oder mit Mycobacterium tuberculosis, wurden nach Klonierung von T-Helfer-Zellen vor allem Klone gefunden, die Lymphotoxin,Gamma-Interferon und Interleukin-2, aber wenig oder kein Interleukin-4, Interleukin-5, Interleukin-6 und Interleukin-10 sezernieren (T-Helfer-Zellen vom sogenannten TH1-Typ).

Nach Infektion von beispielsweise suszeptiblen Mäusen mit parasitären Erregern wie Leishmania major traten bei Klonierungen von T-Helferzellen vor allem Klone auf, die vermehrt Interleukin-4, Interleukin-5 und Interleukin-10 produzieren, aber verringerte oder nicht detektierbare Mengen von Interleukin-2 und Gamma-Interferon (T-Helferzellen vom TH2-Typ) (Mosmann et al., Immunological Reviews 1991, No. 123, 209-229; S. Romagnani, Immunology Today, 256-257, Vol. 12, No. 8 1991).

Dieses vermehrte Auftreten von TH2-Lymphozyten wurde bereits bei einigen Infektionserkrankungen im Tier und im Menschen nachgewiesen (Else and Grencis, Parasitology Today, Vol. 7, No. 11, 1991, pp. 313-316; Parasitology Today, Vol. 7, No. 10, 1991, p. 261) und spiegelt sich auch in sekundären Parametern wieder. So zeigten mit Leishmania major infizierte Mäuse im allgemeinen eine reduzierte Produktion von Gamma-Interferon, stark erhöhtes Serum IgE und Eosinophilie.

Beim Menschen wurde z.B. bei lepromatöser Lepra, Leishmaniasis und Schistosomiasis und Infektionen mit Mycobacterium tuberculosis im allgemeinen stark erhöhte IgE-Konzentration im Serum dieser Patienten im Vergleich zu Seren von Normalpersonen gefunden. Bei den parasitären Infektionen wird oft eine Eosinophilie im Verlauf der Erkrankung beobachtet.

Es wurde nun gefunden, daß Infektionen, die durch ein vermehrtes Auftreten von T-Helferzellen vom TH2-Typ gekennzeichnet sind, mit Hilfe des IL-4R oder von Derivaten davon diagnostiziert, therapiert und/oder prophylaktisch behandelt werden können.

Gegenstand der Erfindung ist daher die Verwendung des IL-4-Rezeptors oder von Derivaten davon zur Herstellung eines Arzneimittels zur Therapie und/oder Prophylaxe oder zur Herstellung eines Diagnostikums zum Nachweis von Infektionen, bei denen T-Helferzellen vom TH2-Typ vermehrt auftreten.

Unter "Derivate" des IL-4R im Sinne der Erfindung versteht man funktionell äquivalente Teile, Mutanten oder Varianten des IL-4R, insbesondere den extrazellulären Teil des IL-4R, vor allem von Aminosäure 1 bis etwa 209 des reifen Proteins des humanen IL-4-Rezeptors aber auch Glykosylierungsmutanten davon. Für die erfindungsgemäße Verwendung des Rezeptors können auch Fusionsproteine eingesetzt werden, die den IL-4R oder Derivate davon sowie andere Proteine bzw. Proteinteile enthalten, vorzugsweise den Fc-Teil von Antikörpern (IL-4R/Fc-Fusionsprotein). Ferner können der IL-4R, Derivate oder Fusionsproteine davon in Kombinationspräparaten zur Diagnose, Therapie und/oder prophylaktischen Behandlung der genannten Krankheiten eingesetzt werden, vorzugsweise in Kombination mit Gamma-Interferon.

Auch ist eine Therapie in Kombination mit gereinigten Allergenen oder Teilen davon, von Vorteil.

Weiterhin von Vorteil ist eine Therapie in Kombination mit Gamma-Interferon und/oder Substanzen, welche die Interaktion des zellulären Oberflächenmoleküls CD40 mit seinem Liganden, dem zellulären Oberflächenmolekül CD40-Ligand, blockieren, vorzugsweise eine lösliche Variante des CD40 Oberflächenmoleküls selbst, wie z. B. ein CD40/Ig-Fusionsprotein entsprechend untenstehender Beschreibung oder Derivaten davon.

Unter "Derivate" einer löslichen Variante des CD40-Oberflächenmoleküls im Sinne der Erfindung versteht man solche funktionell äquivalenten Teile oder Varianten, welche die Interaktion des zellulären CD40 mit dem zellulären Oberflächenmolekül CD40-Ligand blockieren.

Zu den Krankheiten zählen Infektionen, insbesondere virale, bakterielle und parasitäre Infektionen sowie Pilzinfektionen; vorzugsweise Infektionen mit dem Humanen Immundefizienzvirus (HIV), Mycobakterien vor allem Mycobakterium Leprae, mit Listerien, mit Protozoen, vor allem der Gattungen Leishmania und Plasmodium, mit Helminthen, vor allem der Gattungen Schistosoma, Nippostrongylus und Heligmosomoides mit Trichurida, Trichinella, Taenia (Cysticercus), Candida und Aspergillus.

Die Applikationsformen sind im allgemeinen bei unterschiedlichen Erkrankungen verschieden. So kann beispielsweise die topische Applikation bei einigen Erkrankungen von Vorteil sein. Zum Beispiel ist bei Conjunctivitis eine Applikation in Augentropfen, vorteilhaft, da die pathologischen TH2-Zellen vor allem topisch nachgewiesen werden können.

Es wurde beschrieben, daß der menschliche IL-4-Rezeptor aus insgesamt 825 Aminosäuren besteht (Idzerda, R.J. et al. (1990) J. Exp. Med. 171, 861-873). Nach Idzerda et al. fungieren die N-terminalen 25 Aminosäuren als Signalpeptid, der reife humane IL-4-Rezeptor besteht aus 800 Aminosäuren und besitzt eine Dreidomänenstruktur bestehend aus 1. extrazelluläre Domäne (207 Aminosäuren), 2. Transmembranregion (ca. 24 Aminosäuren), 3. cytoplasmatische Domäne (569 Aminosäuren). Eine gentechnische Herstellung des IL-4-Rezeptors ist besonders vorteilhaft, da damit die für die Therapie erforderlichen Substanzmengen ohne weiteres hergestellt werden können.

Die Herstellung des IL-4R kann beispielsweise nach der internationalen Patentanmeldung WO90/05183 erfolgen. Danach wurde eine cDNA-Genbank von beispielsweise der T-Zellinie T22 hergestellt und mit einer Probe auf IL-4R-spezifische DNAs abgesucht. Als Probe kann die in der WO90/05183 beschriebene hybrid-subtrahierte cDNA einer Mauszellinie verwendet werden. Es kann jedoch auch eine IL-4R-spezifische Hybridisierungsprobe, beispielsweise eine oder mehrere ca. 20 Nukleotide lange Proben von beispielsweise Position 193 - 210 zum Absuchen der cDNA-Bank verwendet werden. Nach dem Überprüfen von positiven Klonen auf IL-4R spezifische cDNA z.B. durch Sequenzierung, kann die gefundene IL-4R DNA in geeignete Expressionsvektoren, z.B. pCAV/NOT (WO90/05183) kloniert und in geeigneten Wirtszellen, z.B. C0S-7; BHK-21-, oder CHO-Zellen vollständig oder in Teilen exprimiert werden. Wird nur der für die extrazelluläre Region des IL-4-Rezeptors kodierende Anteil der cDNA für die Expression verwendet, wird die extrazelluläre Region des Interleukin-4 Rezeptors im allgemeinen von transfizierten Zellen in das Kulturmedium ausgeschleust. Hierfür wird die cDNA durch gentechnologische Methoden, die dem Stand der Technik entsprechen, so verändert, daß nach der kodierenden Sequenz für die extrazelluläre Region eines Rezeptors, bzw. gewünschten Anteilen davon, ein Stoppkodon eingeführt wird und die solchermaßen veränderte cDNA in geeignete Expressionsvektoren kloniert wird (Maliszewski et al. (1990), J. Immunol. 144, 3028-3033; Dower et al. (1989), J. Immunol. 142, 4314). In vielen Fällen, so z. B. beim murinen IL-4-Rezeptor, wurden cDNAs isoliert, die für eine natürlicherweise vorkommende sezernierte Form des Rezeptors kodieren (Mosley et al. (1989), Cell 59, 335). Diese cDNAs können dann direkt für die Herstellung von Expressionsvektoren verwendet werden. Die sezernierten Proteine können aus dem Kulturmedium beispielsweise über Liganden-Affinitätschromatographie oder Immuno-Affinitätschromatographie unter Verwendung spezifischermonoklonaler Antikörper gereinigt werden (Maliszewski et al. (1990), J. Immunol. 144, 3028-3033). Wie in der Europäischen Patentanmeldung EP-A1-0 464 533 beschrieben, können ferner auf gentechnologischem Weg Fusionsproteine zwischen dem IL-4R oder Derivaten davon mit anderen Proteinen, beispielsweise mit Immunglobulin-Anteilen, z.B. dem Fc-Teil von Antikörper-Molekülen, hergestellt und zur Expression gebracht werden (im folgenden als IL-4R/Fc bezeichnet). Der Vorteil derartiger Fusionsproteine liegt in einer Verlängerung der Halbwertszeit, und einer vereinfachten Anreicherung und Reinigung über Protein A-Sepharose Affinitätschromatographie.

Wegen der problemlosen Reinigung durch Affinitätschromatographie und der eventuell verbesserten pharmakokinetischen Eigenschaften ist die Synthese von löslichen Formen des IL-4-Rezeptors als Immunglobulin-Fusionsprotein besonders vorteilhaft.

CD40 ist ein Membranprotein des Typ I, d. h. es besteht aus einer (aminoterminalen) extrazellulären Region, einer Transmembranregion und einer (carboxyterminalen) cytoplasmatischen Region. cDNA, die für CD40 kodiert, läßt sich beispielsweise aus einer cDNA-Bank von Raji-Zellen mittels der "Panning"-Methode isolieren (Stamenkovic, I. et al. (1989) EMBO J., Vol. 8, pp. 1403-1410). Um das normalerweise membranständige Protein als lösliche Form zur Expression zu bringen, existieren verschiedene, dem Fachmann wohlbekannte Möglichkeiten. Beispielsweise kann in einer Polymerasekettenreaktion unter Verwendung mutagenisierender Primer ein Stopcodon an das Ende der für die extrazelluläre Region kodierenden cDNA-Sequenz eingeführt werden. Die solcherart veränderte cDNA kodiert dann für ein CD40-Protein, das aufgrund des fehlenden Membranankers wie andere sekretorische Proteine von der Zelle sezerniert wird (Fanslow, W.C. et al. (1992) J. Immunol., pp. 655-660). Vorteilhaft kann auch die Verwendung von löslichen rekombinanten Immunglobulinfusionsproteinen sein, deren Herstellung beispielhaft in EP-A-0 464 533 beschrieben ist. Insbesondere sind CD40/Ig-Fusionsproteine auch aus der Literatur bekannt (Fanslow et al. (1992) J. Immunol., Vol. 149, pp. 655-660 und Noelle et al. (1992) Proc. Natl. Acad. Sci USA, Vol. 89, pp. 6550-6554). In beiden Publikationen werden CD40/Ig-Fusionsproteine beschrieben, bestehend aus der extra-zellulären Region von CD40 fusioniert an Hinge-, CH2- und CH3-Domänen der schweren Kette eines menschlichen Immunglobulin G1-Moleküls. Für die Herstellung der entsprechenden DNA-Konstrukte wurden geeignete Restriktionsschnittstellen mittels Polymerasekettenreaktion unter Verwendung mutagenisierender Primer in die CD40-cDNA eingeführt (Fanslow et al., (1992) J. Immunol., Vol. 149, pp. 655-660) bzw. natürlicherweise vorkommende Restriktionsschnittstellen in der CD40-cDNA genutzt (Noelle et al., (1992) Proc. Natl. Acad. Sci USA, Vol. 89, pp. 6550-6554).

Lösliche Formen von CD40 können in bekannten prokaryotischen oder eukaryotischen Systemen, vorzugsweise jedoch in Säugerzellkulturen, als rekombinante Proteine exprimiert werden und aus Kulturüberständen bzw. Zellaufschlüssen mittels konventionioneller Methoden gereinigt werden. Abgesehen von einem möglichen direkten therapeutischen Einsatz der löslichen CD40-Moleküle, können diese darüber hinaus auch zur Identifizierung von anderen Substanzen eingesetzt werden, die die Interaktion von membranständigem CD40 und CD40-Liganden blokkieren und damit ebenfalls therapeutisches Potential aufweisen. Dies kann beispielsweise in zellfreien Rezeptorbindungstesten geschehen (EP-A-0 488 170), in denen die löslichen CD40-Moleküle auf eine feste Phase verbracht werden und die Bindung von löslichem CD40-Liganden mittels geeigneter Markierung bzw. Antikörper verfolgt wird. Solche Teste bieten wegen ihrer Automatisierbarkeit die Möglichkeit, eine große Anzahl von Substanzen bezüglich ihrer Interaktion mit CD40/CD40-Liganden zu untersuchen ("Rezeptorscreening").

Für die nachstehend aufgeführten Beispiele wurden die in Idzerda et al. (1990, J. Exp. Med. 171, 861-873) bzw. Maliszewski et al. (1990, J. Immunol. 144, 3028-3033) definierten extrazellulären Regionen bzw. natürlicherweise vorkommenden löslichen Formen von humanem bzw. murinem IL-4-Rezeptor (im folgenden als huIL-4R bzw. muIL-4R bezeichnet) verwendet, die nach Doppelselektion mit Methotrexat und G418 (EP-A 0330 977) von stabil exprimierenden BHK-Zellen als lösliches Protein in das Kulturmedium sezerniert und über Immuno-Affinitätschromatographie gereinigt wurden. Darüber hinaus wurden Rezeptor/ Immunglobulin-Fusionsproteine (EP-A1-0 464 533) verwendet, die aus der extrazellulären Region von humanem bzw. murinem IL-4-Rezeptor mit Hinge-, CH2- und CH3-Domänen eines humanen IgGl-bzw. murinen IgG2b-Moleküls (Zettlmeißl et al. (1990) DNA and Cell Biol. 9, 347-353) bestehen (im folgenden als huIL-4R/Fc bzw. muIL-4R/Fc bezeichnet) und ebenfalls nach Expression in BHK-Zellen über Protein A-Sepharose-Affinitätschromatographie gereinigt wurden.

IL-4R und IL-4R/Fc neutralisieren im Bioassay mit gleicher Effektivität Interleukin-4 der jeweils homologen Spezies.-(Beispiel 1). Die Hemmung von humanen T-Zell Klonen des TH2-Typs durch huIL-4R/Fc konnte darüberhinaus auch *in vitro* nachgewiesen werden. Das durch diese Klone *in vitro* gebildete IL-4 wurde durch huIL-4R/Fc neutralisiert. Dies hatte eine Reduktion der Proliferation zur Folge. Kontrollklone vom TH1-Typ wurden durch huIL-4R/Fc nicht in ihrem Wachstum beein-flußt (Beispiel 3).

Außerdem wurde gezeigt, daß huIL-4R/Fc *in vitro* die durch IL-4 induzierte und überraschenderweise auch die antigenspezifische (Beispiel 2) Synthese von IgE durch humane mononukleäre Zellen des peripheren Bluts unterdrücken kann.

Überraschenderweise gibt es einen therapeutischen bzw. prophylaktischen Effekt bei Infektionen wie beispielhaft in einem Tiermodell der murinen Listeriose und in einem Tiermodell (Maus) der Cysticercus Crassiceps-Infektion, für den murinen löslichen Interleukin-4 Rezeptors nachgewiesen werden konnte.

### Beispiel 1:

### Biologische Aktivität der humanen und murinen Varianten von IL-4R und IL-4R/Fc.

Die biologische Aktivität wurde in einem Bioassay gemessen. IL-4 bindet streng speziesspezifisch an den IL-4 Rezeptor. Aus diesem Grund wurde eine Zellinie verwendet, die murinen Ursprungs ist und den murinen IL-4 Rezeptor membranständig trägt. Diese Zellinie wurde mit dem vollständigen Gen für humanen Interleukin-4 Rezeptor transfiziert. Murine und humane membranständige Rezeptoren werden gleichzeitig funktionell von dieser Zellinie exprimiert und die Zellinie proliferiert sowohl in Abhängigkeit von murinem, als auch humanem IL-4 (Mosley et al., Cell, Vol. 59, 335-348, October 20, 1989).

Für den Nachweis von muIL-4R und muIL-4R/Fc wurde murines IL-4 verwendet (Tabelle 1). Die Zellinie proliferiert in Abhängigkeit des murinen IL-4 (Tabelle 1). Wird konstant Interleukin-4 eingesetzt und gleichzeitig muIL-4R oder muIL-4R/Fc zugegeben, kommt es zu einer konzentrations-abhängigen Neutralisation von Interleukin-4. Da weniger Interleukin-4 für die Zellinie verfügbar ist, geht die Proliferation zurück. Ein Kontrollprotein mit gleichem Fc-Teil zeigt diesen Effekt nicht. Unterschiede in der konzentrationsabhängigen Inhibition zwischen muIL-4R und muIL-4R/Fc in Tabelle 1 sind auf die unterschiedlichen Molekulargewichte zurückzuführen.

In Tabelle 2 ist die Bioaktivität von humanem Interleukin-4 und, entsprechend Tabelle 1, der spezifische neutralisierende Effekt von huIL-4R und huIL-4R/Fc dargestellt.
Unterschiede in der konzentrationsabhängigen Inhibition zwischen huIL-4R und huIL-4R/Fc sind auf die unterschiedlichen Molekulargewichte zurückzuführen.

### Beispiel 2:

Humane mononukleäre Zellen des peripheren Bluts wurden isoliert. 5000 Zellen pro Well einer 96-Well Kulturplatte wurden mit 200 *µ*l Iscove's Kulturmedium mit 10 % FKS 14 Tage kultiviert. Der Kultur wurde am Beginn kein weiterer Zusatz, oder 100 SQ-Einheiten (nach Angabe des Herstellers) von gereinigtem Milbenantigen (Derm. Pt., Bestell-Nr. Sq 503, Fa. Scherax, Hamburg) alleine, oder Milbenantigen und 3 *µ*g/ml huIL-4R/Fc zugesetzt. Als Fc-Kontrolle diente ein Ansatz mit Milbenantigen und 3 *µ*g/ml TW1. TW1 ist ein humaner monoklonaler Antikörper mit Spezifität für Rabies-Antigen, der den gleichen Isotyp aufweist wie huIL-4R/Fc. TW1 wurde hergestellt wie in EP-0 445 625 A1 beschrieben. Danach wurde Überstand abgenommen und auf humanes IgE im ELISA getestet. Es wurde gefunden, daß die antigeninduzierte Produktion von IgE durch huIL-4R/Fc spezifisch unterdrückt wird (Tabelle 3).

### Beispiel 3:

### Hemmung der antigen-induzierten Proliferation von T-Zell Klonen des TH2-Subtyps.

T-Zell Klone wurden aus der Haut von Patienten mit atopischer Dermatitis isoliert. Ein hoher Anteil der Klone sezernierte nach Stimulation *in vitro* Cytokine des TH2-Typs, nur ein geringer Anteil an Klonen des TH1-Typs wurde gefunden. Die Proliferationstests wurden in 96-well Mikrotiterplatten mit 1x10⁴ klonierten T-Zellen und 1x10⁵ bestrahlten autologen PBL als Stimulatorzellen in Iscove's Kulturmedium mit 4 % humanem AB-Serum durchgeführt. Die Kultur wurde 18 h mit 0,5 *µ*Ci/Kulturvertiefung ³H-Thymidin im CO₂-begasten Brutschrank bei 37°C durchgeführt. Nach 18 Stunden wurde die Proliferation der Zellen anhand des eingebauten Thymidins bestimmt. Für die Produktion von Cytokinen im Überstand wurden 1x10⁶/ml klonierte T-Zellen mit Iscove, 10 % FCS und 10 *µ*g/ml Concanavalin-A in 24 well Kulturplatten stimuliert. Die Überstände wurden nach 24 h Kulturdauer im CO₂-begasten Brutschrank (37°C) abgenommen. Die Überstände der Kulturen wurden in Bioassays auf den Gehalt an humanem Interleukin-2 und humanem Interleukin-4 getestet. Die Bioassays basieren auf Zelllinien, die abhängig von humanem Interleukin-2 bzw. humanem Interleukin-4 proliferieren (Mosley et al. Cell, Vol. 59, 335-348, October 20, 1989). In den Überständen eines TH2-Klons konnte kein IL-2, aber viel IL-4 nachgewiesen werden. Bei Zugabe von 3 *µ*g/ml von huIL-4R/Fc konnten statt 49 ng/ml IL-4 nur noch 10 ng/ml IL-4 nachgewiesen werden. Überstände eines TH1-Klons enthielten vergleichsweise viel IL-2, aber nur wenig IL-4. Die Interleukin-2 Konzentration in den Überständen des TH1-Klons wird nicht signifikant durch Zugabe von huIL-4R/Fc während der Kultur beeinflußt. Im Proliferationsexperiment konnte nachgewiesen werden, daß die Proliferation des TH2-Klons, nicht aber des TH1-Klons durch Zugabe von 3 *µ*g/ml huIL-4R/Fc unterdrückt wird (Tabelle 4). Zum weiteren konnte gezeigt werden, daß durch Zugabe von huIL-4R/Fc die antigenspezifische Proliferation milbenspezifischer TH2-Klone, nicht aber TH1-Klone unterdrückt wird. In einem weiteren Versuchsansatz konnte nachgewiesen werden, daß die durch Milbenantigen induzierte IgE-Synthese eines Gemisches von einem TH2-Klon mit autologen B-Lymphozyten desselben Spenders durch Zugabe von huIL-4R/Fc unterdrückt werden kann.

### Beispiel 4:

### Behandlung einer Infektion mit Cysticercus (Taenia) crassiceps in der Maus mit murinem Interleukin-4 Rezeptor

Für das Experiment wurden am Tag 0 NMRI-Mäuse mit 15-25 g Körpergewicht mit 5 einzelnen Metacestoden per Maus in 1 ml PBS i.p. des Cysticercus (Taenia) crassiceps Stammes MR-1 infiziert. Es wurden pro Experimentalgruppe 10 Tiere eingesetzt. Eine Gruppe wurde zur Kontrolle nicht infiziert (Injektion von PBS ohne-Metacestoden). Eine Gruppe wurde infiziert, aber nicht behandelt. Eine Gruppe wurde infiziert und an den Tagen -1, 0, 1, 7, 14, 20, 30, 35, 40, 45, 50, 55 und 63 mit jeweils 100 *µ*g i.p. muIL-4R behandelt. Eine weitere Gruppe wurde infiziert und analog der Behandlung mit muIL-4R mit huIL-4R als Kontrollprotein behandelt. Am Tag 76 wurden die Tiere gewogen und eine Sektion durchgeführt. Es wurde bestimmt, bei welchen Tieren Metacestoden im Bauchraum gefunden werden konnten. Bei den positiven Tieren wurde das Naßgewicht der gefundenen Metacestoden pro Tier bestimmt. Bei der Gruppe mit Behandlung mit murinem Interleukin-4 Rezeptor konnte eine deutliche Reduktion des Gesamtgewichts der Metacestoden gefunden werden (Tabelle 5).

### Beispiel 5:

### Einfluß von muIL-4R auf die murine Candida albicans Infektion

Weibliche Hybrid-Mäuse (BALB/cCrxDBA/2Cr)F1 (CD2F1) wurden mit dem hoch pathogenen Candida albicans Stamm CA-6 (5 x 10⁴ Zellen intravenös) infiziert (Tag 0 des Versuchs). Beginnend 24 Stunden vor der Infektion erhielten die Tiere je 100 *µ*g muIL-4R in 250 *µ*l PBS intraperitoneal einmal am Tag -1, je zweimal am Tag 0 und 1 und je einmal am Tag 2 und 3. Kontrolltiere erhielten alternativ mit dem gleichen Schema gereinigtes Mausserumalbumin (MSA), huIL-4R oder PBS. Alle Tiere, die mit Mausserumalbumin (16 Tiere), huIL-4R (16 Tiere) und PBS (24 Tiere) behandelt wurden, entwickelten eine progressive Candida-Infektion mit mittleren Überlebenszeiten von 14-17 Tagen nach Infektion. Von den Tieren, die mit muIL-4R behandelt wurden (24 Tiere) überlebten 91,6 %. Diese überlebenden Tiere wurden 8 Wochen nach Infektion erneut mit Candida albicans (10⁶ Zellen) infiziert. Alle Tiere überlebten diese erneute Infektion. Diese Infektionsdosis führt bei normalen, unbehandelten Tieren zu einer letalen Infektion mit Überlebenszeiten von 2-3 Tagen nach Infektion.

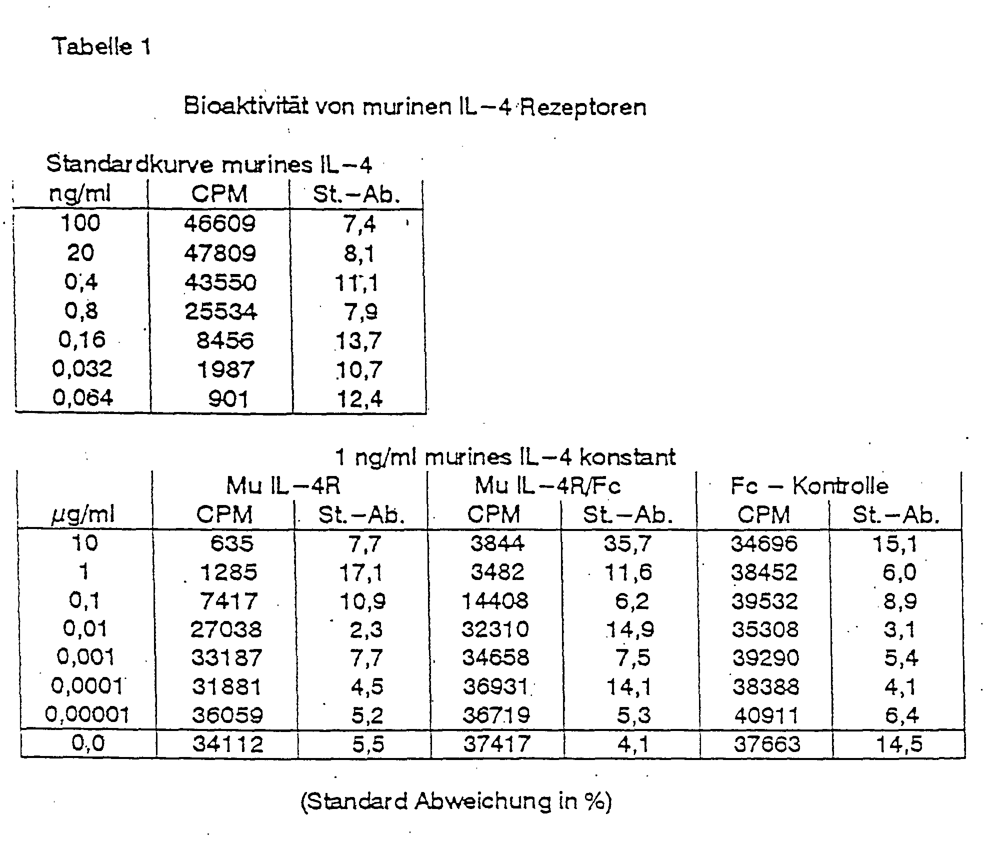

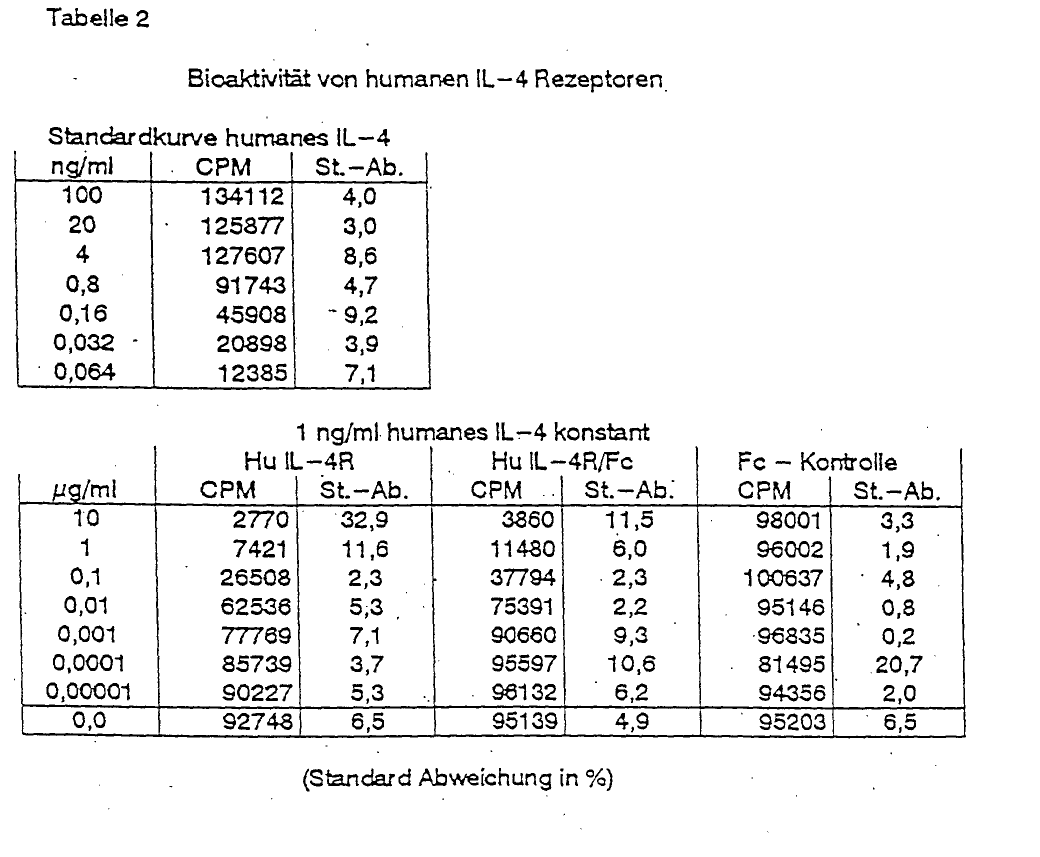

**Tabelle 3**

| Inhibition der Synthese von allergenspezifischem Ig E | | | |
|---|---|---|---|
| Medium | 100 SQ E/ml Milben Allergen Derm. Pt. | | |
| Kontrolle | ohne hu IL-4R/Fc | 3 *µ*g/ml hu IL-4R/Fc | 3 *µ*g/ml Fc - Kontrolle |
| <0,09 | 1,23 (4,1) | <0,09 | 1,17 (5,6) |
| Angegeben ist humanes IgE in ng/ml. | | | |
| In Klammern: prozentuale Standardabweichung | | | |

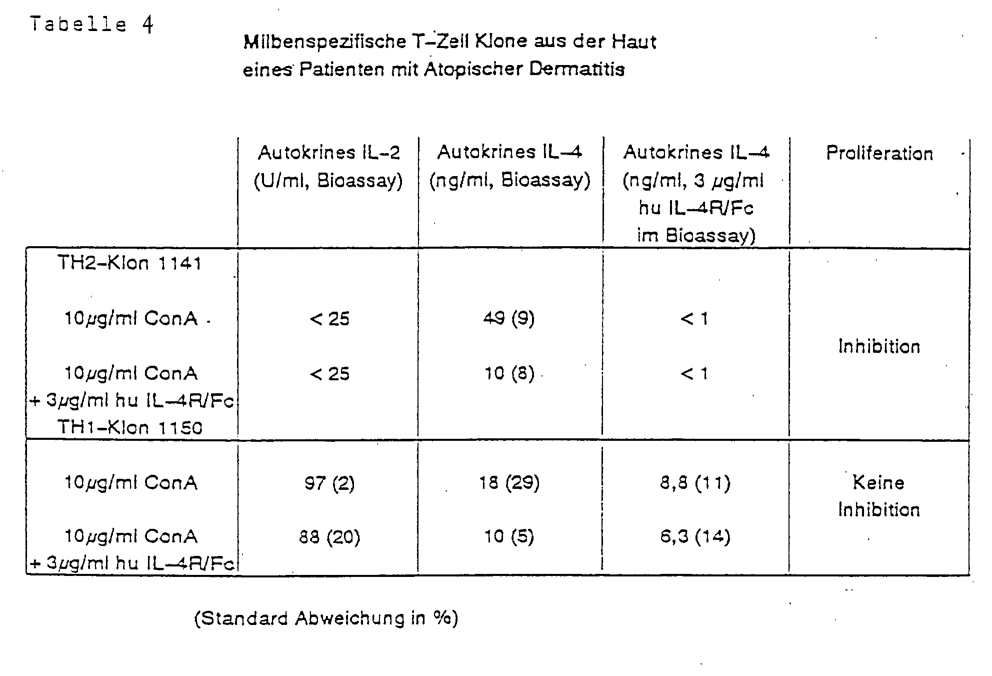

**Tabelle 5**

| Behandlung einer Infektion mit Cysticercus (Taenia) crassiceps in der Maus mit murinem Interleukin-4 Rezeptor | | | | |
|---|---|---|---|---|
| Behandlung mit | Körpergewicht (Gramm) | Parasitenlast (Gramm Naßgewicht pro Einzeltier | | Parasitenlast (Gramm Naßgewicht pro Gruppe |
| muIL-4R | 35.7 | neg | neg | |
| | | 0.1 | neg | |
| | | 6.0 | 7.5 | 13.7 |
| | | neg | 0.1 | |
| | | neg | neg | |
| huIl-4R | 35.9 | < 0.1 | neg | |
| | | < 0.1 | neg | |
| | | 8.4 | 6.3 | 34.9 |
| | | 8.2 | 5.7 | |
| | | 1.3 | 5.0 | |
| Infektions-kontrolle | 35.8 | 1.0 | 5.8 | |
| | | neg | 3.7 | |
| | | 2.7 | 2.1 | 31.6 |
| | | 7.2 | 6.2 | |
| | | 1.0 | 1.9 | |
| O-Kontrolle | 34.5 | - | - | |

## Patentansprüche

1. Verwendung des IL-4-Rezeptors (IL-4R) oder eines Derivates davon zur Herstellung eines Arzneimittels zur Therapie und Prophylaxe von Infektionen, die dadurch gekennzeichnet sind, daß T-Helferzellen vom TH2-Typ vermehrt auftreten.

2. Verwendung des IL-4R oder eines Derivates davon zur Herstellung eines Diagnostikums zum Nachweis von Infektionen, die dadurch gekennzeichnet sind, daß T-Helferzellen vom TH2-Typ vermehrt auftreten.

3. Verwendung des IL-4R oder eines Derivates davon nach Anspruch 1, dadurch gekennzeichnet, daß die Derivate Teile, Mutanten oder Varianten des IL-4R sind.

4. Verwendung des IL-4R oder eines Derivates davon nach Anspruch 3, dadurch gekennzeichnet, daß ein Derivat die extrazelluläre Region des IL-4R ist.

5. Verwendung des IL-4R oder eines Derivates davon nach Anspruch 1, dadurch gekennzeichnet, daß der IL-4-Rezeptor oder ein Derivat davon Bestandteil eines Fusionsproteins ist.

6. Verwendung des IL-4R oder eines Derivates davon nach Anspruch 5, dadurch gekennzeichnet, daß das Fusionsprotein den IL-4-Rezeptor oder ein Derivat davon und den Fc-Teil eines Antikörpers enthält.

7. Verwendung des IL-4R oder eines Derivates davon nach einem oder mehreren der Ansprüche 1-6, dadurch gekennzeichnet, daß die Infektionen virale, bakterielle, parasitäre Infektionen oder Pilzinfektionen sind.

8. Verwendung des IL-4R oder eines Derivates davon nach einem oder meheren der Ansprüche 1-6, dadurch gekennzeichnet, daß es sich bei den Infektionen um Infektionen mit Retroviren, Plectomyceten, Hefepilzen, Mykobakterien, Listerien, Protozoen, Cestoden oder Helminthen handelt.

9. Verwendung des IL-4R oder eines Derivates davon nach einem oder mehreren der Ansprüche 1-6, dadurch gekennzeichnet, daß es sich bei den Infektionen um Infektionen mit dem Humanen Immundefizienzvirus (HIV), Mycobakterium leprae, Leishmania, Plasmodium, Schistosoma, Nippostrongylus, Trichurida, Trichinella, Taenia (Cysticercus), Candida, Aspergillus, Cyclophilida oder Heligmosomoides handelt.

10. Verwendung des IL-4R oder eines Derivates davon nach einem oder mehreren der Ansprüche 1-6, dadurch gekennzeichnet, daß der IL-4R oder ein Derivat davon in einem Kombinationspräparat verwendet wird.

11. Verwendung des IL-4R oder eines Derivates davon nach Anspruch 10, dadurch gekennzeichnet, daß das Kombinationspräparat Gamma-Interferon enthält.

12. Verwendung des IL-4R oder eines Derivates davon nach Anspruch 10, dadurch gekennzeichnet, daß das Kombinationspräparat Allergene oder Teile von Allergenen enthält.

13. Verwendung des IL-4R oder eines Derivates davon nach Anspruch 10, dadurch gekennzeichnet, daß das Kombinationspräparat eine oder mehrere Substanzen enthält, welche die Interaktion des zellulären Oberflächenproteins CD40-Ligand mit dem zellulären Oberflächenprotein CD40 inhibieren.

14. Verwendung des IL-4R oder eines Derivates davon nach Anspruch 13, dadurch gekennzeichnet, daß mindestens eine der Substanzen eine lösliche Determinante des CD40-Oberflächenmoleküls oder eines Derivates davon darstellt.

15. Verwendung des IL-4R oder eines Derivates davon zur Herstellung eines Arzneimittels gemäß einem oder mehreren der Ansprüche 1-6 zur topischen Applikation.

16. Verwendung des IL-4R oder eines Derivates davon zur Herstellung eines Arzneimittels gemäß Anspruch 15 zur intradermalen, subcutanen, dermalen, nasalen oder pulmonalen Applikation.

## Claims

1. The use of the IL-4 receptor (IL-4R) or of a derivative thereof for the production of a pharmaceutical for the therapy and prophylaxis of infections in which there is increased occurrence of T-helper cells of the TH2 type.

2. The use of IL-4R or of a derivative thereof for producing a diagnostic aid for identifying infections in which there is increased occurrence of T-helper cells of the TH2 type.

3. The use of IL-4R or of a derivative thereof as claimed in claim 1, wherein the derivatives are parts, mutants or variants of IL-4R.

4. The use of IL-4R or of a derivative thereof as claimed in claim 3, wherein a derivative is the extracellular region of IL-4R.

5. The use of IL-4R or of a derivative thereof as claimed in claim 1, wherein the IL-4 receptor or a derivative thereof is a constituent of a fusion protein.

6. The use of IL-4R or of a derivative thereof as claimed in claim 5, wherein the fusion protein contains the IL-4 receptor or a derivative thereof and the Fc portion of an antibody.

7. The use of IL-4R or of a derivative thereof as claimed in one or more of claims 1-6, wherein the infections are viral, bacterial, parasitic infections or fungal infections.

8. The use of IL-4R or of a derivative thereof as claimed in one or more of claims 1-6, wherein the infections are infections with retroviruses, plectomycetes, yeast-like fungi, mycobacteria, listeria, protozoa, cestodes or helminths.

9. The use of IL-4R or of a derivative thereof as claimed in one or more of claims 1-6, wherein the infections are infections with the human immunodeficiency virus (HIV), Mycobacterium leprae, Leishmania, Plasmodium, Schistosoma, Nippostrongylus, Trichurida, Trichinella, Taenia (Cysticercus), Candida, Aspergillus, Cyclophilida or Heligmosomoides.

10. The use of IL-4R or of a derivative thereof as claimed in one or more of claims 1-6, wherein IL-4R or a derivative thereof is used in a combination product.

11. The use of IL-4R or of a derivative thereof as claimed in claim 10, wherein the combination product contains gamma-interferon.

12. The use of IL-4R or of a derivative thereof as claimed in claim 10, wherein the combination product contains allergens or parts of allergens.

13. The use of IL-4R or of a derivative thereof as claimed in claim 10, wherein the combination product contains one or more substances which inhibit the interaction of the cellular surface protein CD40 ligand with the cellular surface protein CD40.

14. The use of IL-4R or of a derivative thereof as claimed in claim 13, wherein at least one of the substances is a soluble determinant of the CD40 surface molecule or of a derivative thereof.

15. The use of IL-4R or of a derivative thereof for the production of a pharmaceutical as claimed in one or more of claims 1-6 for topical administration.

16. The use of IL-4R or of a derivative thereof for the production of a pharmaceutical as claimed in claim 15 for intradermal, subcutaneous, dermal, nasal or pulmonary administration.

## Revendications

1. Utilisation du récepteur d'IL-4 (IL-4R) ou d'un dérivé de celui-ci pour la préparation d'un médicament pour la thérapie et la prophylaxie d'infections qui sont caractérisées en ce que des cellules T auxiliaires du type TH2 apparaissent de manière amplifiée.

2. Utilisation d'IL-4R ou d'un dérivé de celui-ci pour la préparation d'un agent de diagnostic pour la mise en évidence d'infections qui sont caractérisées en ce que des cellules T auxiliaires du type TH2 apparaissent de manière amplifiée.

3. Utilisation d'IL-4R ou d'un dérivé de celui-ci selon la revendication 1, caractérisée en ce que les dérivés sont des parties, des mutants ou des variantes d'IL-4R.

4. Utilisation d'IL-4R ou d'un dérivé de celui-ci selon la revendication 3, caractérisée en ce qu'un dérivé est la région extracellulaire d'IL-4R.

5. Utilisation d'IL-4R ou d'un dérivé de celui-ci selon la revendication 1, caractérisée en ce que le récepteur d'IL-4 ou un dérivé de celui-ci fait partie d'une protéine de fusion.

6. Utilisation d'IL-4R ou d'un dérivé de celui-ci selon la revendication 5, caractérisée en ce que la protéine de fusion contient le récepteur d'IL-4 ou un dérivé de celui-ci et la partie Fc d'un anticorps.

7. Utilisation d'IL-4R ou d'un dérivé de celui-ci selon une ou plusieurs des revendications 1 à 6, caractérisée en ce que les infections sont des infections virales, bactériennes, parasitaires ou des infections fongiques.

8. Utilisation d'IL-4R ou d'un dérivé de celui-ci selon une ou plusieurs des revendications 1 à 6, caractérisée en ce que les infections sont des infections avec des rétrovirus, des plectomycètes, des levures, des mycobactéries, des listeria, des protozoaires, des cestodes ou des helminthes.

9. Utilisation d'IL-4R ou d'un dérivé de celui-ci selon une ou plusieurs des revendications 1 à 6, caractérisée en ce que les infections sont des infections avec le virus de l'immunodéficience humaine (VIH), Mycobacterium leprae, Leishmania, Plasmodium, Schistosoma, Nippostrongylus, Trichurida, Trichinella, Taenia (Cysticercus), Candida, Aspergillus, Cyclophilida ou Heligmosomoides.

10. Utilisation d'IL-4R ou d'un dérivé de celui-ci selon une ou plusieurs des revendications 1 à 6, caractérisée en ce que l'IL-4R ou un dérivé de celui-ci est utilisé dans une préparation de combinaison.

11. Utilisation d'IL-4R ou d'un dérivé de celui-ci selon la revendication 10, caractérisée en ce que la préparation de combinaison contient de l'interféron gamma.

12. Utilisation d'IL-4R ou d'un dérivé de celui-ci selon la revendication 10, caractérisée en ce que la préparation de combinaison contient des allergènes ou des parties d'allergènes.

13. Utilisation d'IL-4R ou d'un dérivé de celui-ci selon la revendication 10, caractérisée en ce que la préparation de combinaison contient une ou plusieurs substances qui inhibent l'interaction de la protéine de surface cellulaire ligand de CD40 avec la protéine de surface cellulaire CD40.

14. Utilisation d'IL-4R ou d'un dérivé de celui-ci selon la revendication 13, caractérisée en ce qu'au moins l'une des substances représente un déterminant soluble de la molécule de surface CD40 ou d'un dérivé de celle-ci.

15. Utilisation d'IL-4R ou d'un dérivé de celui-ci pour la préparation d'un médicament selon une ou plusieurs des revendications 1 à 6 pour l'application topique.

16. Utilisation d'IL-4R ou d'un dérivé de celui-ci pour la préparation d'un médicament selon la revendication 15 pour l'application intradermique, sous-cutanée, dermique, nasale ou pulmonaire
